# EUROPÄISCHE PATENTANMELDUNG

(11) **EP 3 130 380 A1**
(43) Veröffentlichungstag der Anmeldung: **15.02.2017**
(21) Anmeldenummer: 15401086.2
(22) Anmeldetag: 12.08.2015
(51) Int. Cl.: A63B 69/00, G02C 7/16, A61F 9/04, A63B 102/16

(54) **VORRICHTUNG ZUR BEGRENZUNG DES SICHTFELDES EINES MENSCHEN**

(71) Anmelder: Tuncer, Abbdurrahman, 68333 Völklingen (DE)
(72) Erfinder: Tuncer, Abbdurrahman, 68333 Völklingen (DE)
(74) Vertreter: Sartorius, Peter

(57) **Zusammenfassung**

Die Erfindung betrifft eine Vorrichtung zur Begrenzung des Sichtfeldes eines Menschen mit Hilfe eines am Körper oder am Kopf zu befestigenden Tragteils.

Der Erfindung liegt die Aufgabe zugrunde, die Vorrichtung zur Begrenzung des Sichtfeldes eines Menschen auf einfache und kostengünstige Weise herzustellen und das Sichtfeld so zu begrenzen, dass die Person beim sportlichen Einsatz ihren Blick hauptsächlich auf sein Gegenüber richtet und mit Hilfe der Vorrichtung daran gehindert wird, auf ihre Füße zu schauen.

Gelöst wird die Aufgabe erfindungsgemäß dadurch, dass das Tragteil (2) eine Einrichtung aufweist, mit deren Hilfe das Sichtfeld des Menschen auf einen Ausschnitt eingeschränkt wird.

## Beschreibung

Die Erfindung betrifft eine Vorrichtung zur Begrenzung des Sichtfeldes eines Menschen mit Hilfe eines am Körper oder am Kopf zu befestigenden Tragteils.

Es ist bereits allgemein bekannt, bei einer Brille ein Tragteil zu verwenden, das lediglich die Aufgabe hat, Brillengläser aufzunehmen.

Der Erfindung liegt die Aufgabe zugrunde, die Vorrichtung zur Begrenzung des Sichtfeldes eines Menschen auf einfache und kostengünstige Weise herzustellen und das Sichtfeld so zu begrenzen, dass die Person u.a. beim sportlichen Einsatz ihren Blick hauptsächlich auf sein Gegenüber richtet und mit Hilfe der Vorrichtung daran gehindert wird, auf ihre Füße zu schauen.

Die Aufgabe wird erfindungsgemäß dadurch gelöst, dass das Tragteil eine Einrichtung aufweist, mit deren Hilfe das Sichtfeld des Menschen auf einen Ausschnitt eingeschränkt wird. Hierdurch wird auf einfache Weise sichergestellt, dass die Person oder der Fußballspieler während des Fußballspiels seinen Blick nicht auf seine Füße ausrichtet, sondern auf den Fußballgegner und dadurch sämtliche Aktionen in kürzester Zeit wahrnimmt und darauf mit der richtigen Maßnahme reagiert. Dabei ist die schnellste Art des Handelns oder des Reagierens der Reflex, der Person, die dem Gegner gegenübersteht von Bedeutung. Es werden Reaktionszeiten von 0,025-0,04 s erreicht. An den Augen, der Körperhaltung und an weiteren nonverbalen Gesten kann das Gehirn Muster erkennen und reflexartig vorhersagen, welche Bewegung der Ball machen wird. Würde der Fußballspieler, wie bisher üblich, seinen Blick nach unten ausrichten und unter anderem auf seine Füße blicken und erst danach den Gegner ins Visier nehmen, verlöre er wertvolle Zeit, die er benötigt, alle Aktionen des Gegner wahrzunehmen. An den Augen, der Körperhaltung und an weiteren nonverbalen Gesten kann das Gehirn Muster erkennen und reflexartig vorhersagen, welche Bewegung der Ball machen wird. Dies erlebt man beim Tischtennis, dass der Ball so schnell die Plattenseite wechselt, dass der Mensch nicht aktiv darüber nachdenken kann, wo er als nächstes hinschlagen müsste. Er reagiert reflexartig! Der Blickwinkelwechsel von unten nach oben und vorne in Richtung des Gegners benötigt zwar nur eine sehr kurze Zeit, eventuell Millisekunden, die aber bei einem schnellen Kampfsport von großer Bedeutung sein können. Es ist dabei zu beachten, dass bei einem Kampfsport viele Aktionen im Millisekunden-Bereich ausgeführt werden. Diese Aktionen müssen aber von der angreifenden Person oder dem Fußballspieler erfasst, verarbeitet und mit der richtigen Aktion beantwortet werden. Die sofortige Reaktion sollte mit einer Vorrichtung, die zur Begrenzung des Sichtfeldes beiträgt - trainiert werden, denn die Aktionen der Fußballspieler während eines Fußballspiels soll seine Blicke auf den Gegner ausrichten und nicht auf sein Füße. Hinweise des Trainers, der Spieler solle nur den Gegner im Auge haben, helfen wenig, da während des Spiels solche Ermahnungen leicht in Vergessenheit geraten oder im Rahmen der Stressüberflutung vom Fußballspieler nicht wahrgenommen werden. Hier will also die Erfindung Abhilfe schaffen und dafür sorgen, dass der agierende Spieler stets seine Blicke in Richtung des Gegners ausrichtet. Schaut er dennoch nach unten, wird er feststellen, dass er dort nicht sieht, sodass er nach einer gewissen Gewöhnungszeit lernt, nicht nach unten sondern nach vorne in Richtung des Gegners zu schauen.

Hierzu ist es vorteilhaft, dass das Tragteil als Band, elastisch und/oder längenveränderlich ausgebildet ist und am Kopf des Menschen befestigt werden kann. Mit Hilfe des elastisch und/oder längenveränderlich ausgebildeten Tragteils lässt sich dieses in kürzester Zeit am Kopf in die gewünschte, richtige Position verstellen und festsetzen, sodass es beim Spiel seine Position beibehält. Sollte es dennoch verrutschen, kann die Spannung des Bandes verändert werden und das Tragteil ohne weiteres wieder in die richtige Position zurückverstellt und langhaltig besser gesichert werden.

Eine zusätzliche Möglichkeit ist gemäß einer Weiterbildung der Erfindung, dass das Tragteil aus einem festen Material besteht, das an Teilen des Kopfes oder an der Nase und/oder an den Ohren abstützbar ist, wobei das Tragteil aus einem oder mehreren Teilen besteht, die fest und/oder einteilig miteinander verbunden sind und an unterschiedliche Kopfformen angepasst werden können.

Ferner ist es vorteilhaft, dass zumindest ein Teil oder das vordere Teil ein sich auf dem Nasenrücken abstützendes Formteil aufweist, an das sich an beiden Seitenenden des Formteils je ein weiteres oder ein in etwa flaches Teil anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann.

Vorteilhaft ist es auch, dass an beiden Seitenenden des Abstützteils je ein in etwa flaches Teil anschließt, das mit Bezug auf die Standfläche des Menschen horizontal verläuft oder zumindest horizontal einstellbar ist. Die Verstellmöglichkeit des flachen Teils stellt sicher, dass es immer so ausgerichtet werden kann, dass ein kleiner Bereich der Standfläche des Menschen nicht wahrgenommen werden kann.

Vorteilhaft ist es, dass das flache und/oder horizontal einstellbare Teil, das beiderseits des abstützbaren Teils und/oder des Formteils vorgesehen ist, mit dem abstützbaren Teil und/oder Formteil beweglich und/oder einstellbar und/oder lösbar verbunden ist.

Von besonderer Bedeutung ist für die vorliegende Erfindung, dass das flache und/oder horizontal einstellbare Teil, das beiderseits des abstützbaren Teils und/oder Formteils vorgesehen ist, mit je einem weiteren seitlich am Kopf verlaufenden Teil beweglich und/oder einstellbar und/oder lösbar verbunden ist. Auf diese Weise erhält man eine sehr gute Anpassung an den Kopfumfang des Anwenders.

Auch ist es vorteilhaft, dass sich das seitlich am Kopf verlaufende Teil an eine seitliche Kopffläche anlegt und/oder auf den Ohren des Kopfes abgestützt werden kann.

Ferner ist es vorteilhaft, dass die vorderen Teile des Sichtfeldes eines Menschen verstellbar ausgebildet und/oder miteinander einstellbar verbunden sind, wobei der Querschnitt zumindest einzelner Teile flach, oval oder so ausgebildet sind, dass sie das Sichtfeld des Menschen zumindest zu einer Seite oder in eine Richtung begrenzen, wobei zumindest ein oder mehrere Teile des Tragteils unterhalb der Augen am Kopf des Menschen befestigt werden.

Vorteilhaft ist es auch, dass das am Körper oder am Kopf zu befestigende Tragteil die Kopfform ganz umschließt und der hintere Teil mit Bezug auf den Kopf eines Menschen als einstellbares Teil und/oder elastisch einstellbares und/oder längenveränderliches Teil oder Band ausgebildet ist.

Ferner ist es vorteilhaft, dass einzelne oder alle Teile des am Kopf zu befestigen Tragteils Einzelelemente sind, die mit dem jeweils benachbarten Teil fest und/oder lösbar und/oder beweglich und/oder einstellbar verbunden sind und dass das Tragteil ein weiteres Befestigungsteil aufweist, mit dessen Hilfe das Tragteil noch sicherer am Kopf befestigt werden kann.

Weitere Vorteile und Einzelheiten der Erfindung sind in den Patentansprüchen und in der Beschreibung erläutert und in den Figuren dargestellt.

Dabei zeigen:
- Fig. 1: eine perspektivische Darstellung der Vorrichtung zur Begrenzung des Sichtfeldes eines Menschen mit Hilfe eines Tragteils, das als Materialbogen unterschiedlicher Breite am Kopf eines Menschen befestigt werden kann;
- Fig. 2: eine Draufsicht, gemäß Fig. 1;

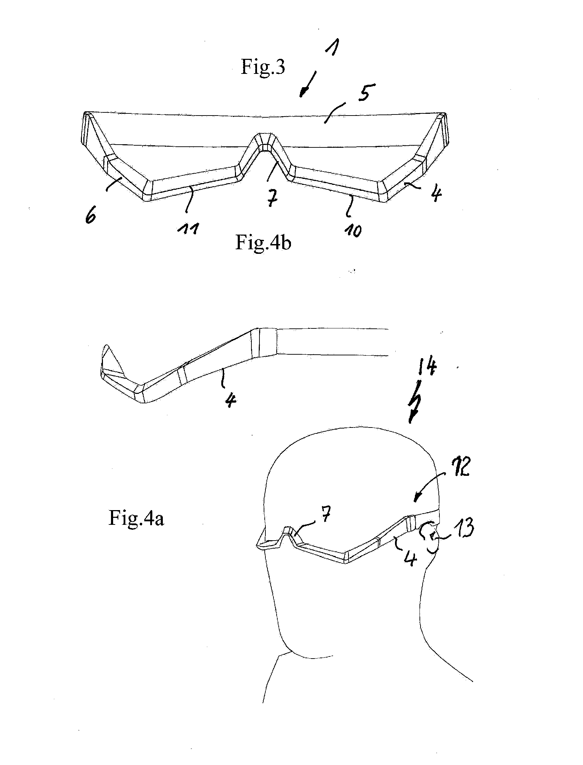

- Fig. 3: eine perspektivische Vorderansicht, gemäß Fig. 1;
- Fig. 4a: das auf dem Kopf eines Athleten oder Fußballspielers aufgesetzte Tragteil;
- Fig. 4b: einen seitlichen Bügel des Tragteils;

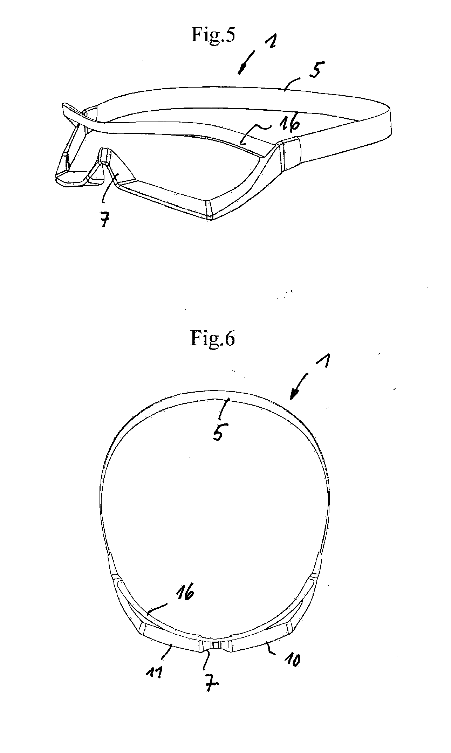

- Fig. 5: ein weiteres Ausführungsbeispiel des Tragteils mit einem oberhalb der Augen vorgesehenen Halteband;
- Fig. 6: eine Draufsicht, gemäß Fig. 5;

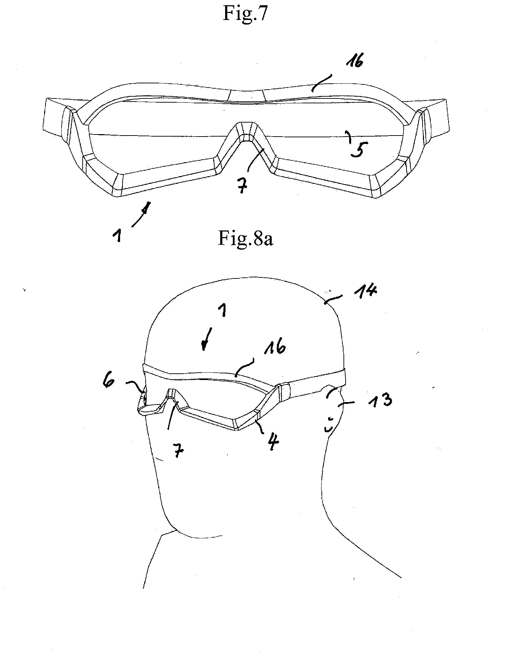

- Fig. 7: eine perspektivische Darstellung in der Ansicht von vorne des Ausführungsbeispiels des Tragteils, gemäß Fig. 5 mit einem oberhalb der Augen vorgesehenen Halteband;
- Fig. 8a: ein auf dem Kopf eines Akteurs aufgesetzte Tragteil, gemäß Fig. 5;

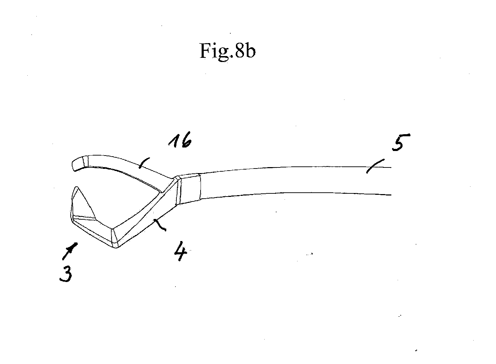

- Fig. 8b: einen seitlichen Bügel des Tragteils, gemäß Fig. 5.

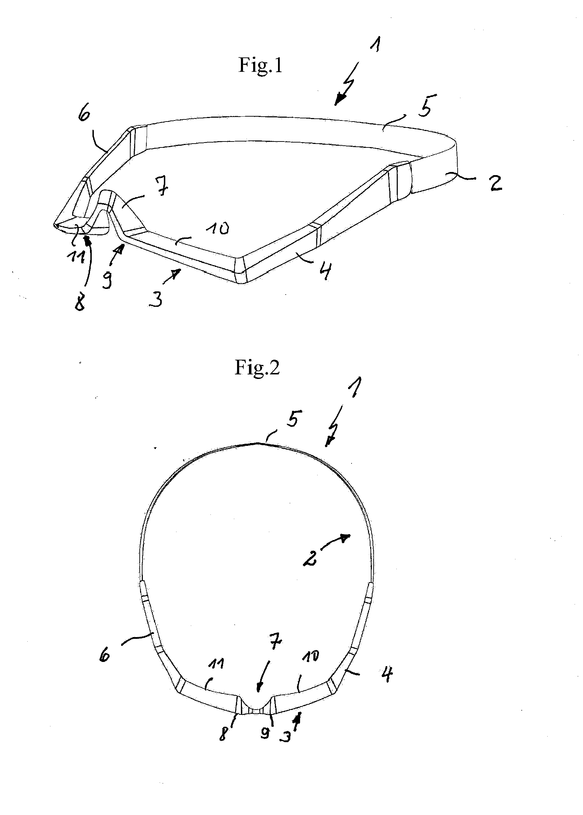

In Fig. 1 ist eine Vorrichtung mit 1 bezeichnet, die eine Einrichtung aufweist, mit deren Hilfe das Sichtfeld des Menschen auf einen Ausschnitt eingeschränkt wird. Der hier erwähnte Mensch kann ein Athlet sein, der gegen einen anderen Athleten im Kampfsport, wie Boxen oder andere Sportarten, antritt. Hierdurch wird auf einfache Weise sichergestellt, dass die Person z. B. während des Fußballspiels ihren Blick nicht auf ihre Füße ausrichtet, sondern auf den Gegner und dadurch sämtliche Aktionen in kürzester Zeit wahrnimmt und darauf mit der richtigen Maßnahme reagiert. Würde der Fußballspieler, wie oft üblich, seinen Blick nach unten ausrichten und unter anderem nur auf seine Füße blicken und erst danach den Gegner ins Visier nehmen, verlöre er wertvolle Zeit, die er benötigt alle Aktionen des Gegner rechtzeitig wahrzunehmen. Der Blickwechsel von unten nach oben und wieder nach vorne in Richtung des Gegners benötigt zwar nur eine sehr kurze Zeit, eventuell Millisekunden, die aber bei schnellen Interaktionen von sehr großer Bedeutung sein können. Es ist dabei zu beachten, dass u.a. bei einem Kampfsport viele Aktionen in Millisekunden Bereich ausgeführt werden. Diese Aktion müssen aber von der angreifenden Person oder des Fußballspielers sofort erfasst, verarbeitet und mit der richtigen Aktion beantwortet werden.

Betrachtet man die Fußballspieler im Einsatz, so kann man häufig beobachten, dass sie während des Fußballspiels ihre Blicke nicht auf den Gegner ausrichten, sondern vielfach auf den Bereich ihrer Füße. Hinweise des Trainers, der Spieler soll nur den Gegner im Auge haben, helfen wenig, da während des Spiels solche Ermahnungen leicht in Vergessenheit geraten. Hier will also die Erfindung Abhilfe schaffen und dafür sorgen, dass der Spieler stets seine Blicke in Richtung des Gegners ausrichtet. Schaut er dennoch nach unten, wird er feststellen, dass er dort nicht sieht, sodass er nach einer gewissen Gewöhnungszeit lernt, nicht nach unten sondern nur nach vorne in Richtung des Gegners zu schauen. Die Sehgewohnheiten lassen sich mit Hilfe eines im Tragteil vorgesehenen Sensors erfassen und an einen Rechner übertragen, um auf diese Weise die Sehgewohnheiten des Spielers zu ermitteln und nach und nach zu korrigieren. Deshalb ist es vorteilhaft, wenn das Tragteil mit der Sehbegrenzung insbesondere zu Trainingszwecken eingesetzt wird.

Hierzu ist das Tragteil 2 als Band 3 ausgebildet, das elastisch und/oder längenveränderlich ausgebildet ist und am Kopf des Menschen befestigt werden kann. Mit Hilfe des elastisch und/oder längenveränderlich ausgebildeten Tragteils 2 lässt sich dieses in kürzester Zeit am Kopf 14 in die gewünschte richtige Position verstellen und festsetzen, sodass es beim Spiel seine Position beibehält. Sollte es dennoch verrutschen, so kann die Spannung des Bandes verändert werden und das Tragteil 2 ohne weiteres wieder in die richtige Position zurückverstellt und langhaltig gesichert wird.

Eine zusätzliche Möglichkeit besteht darin, dass das Tragteil 2 aus einem festen Material besteht, das an Teilen des Kopfes 14 oder an der Nase und/oder an den Ohren 13 abstützbar ist, wobei das Tragteil 2 aus einem oder mehreren Teilen 3, 4, 5, 6 bestehen kann, die fest und/oder einteilig miteinander verbunden sind, um es unterschiedlichen Kopfformen anzupassen.

Ferner ist es vorteilhaft, dass zumindest ein Teil oder das vordere Teil 3 ein sich auf dem Nasenrücken abstützendes Formteil 7 aufweist, an das sich an beiden Seitenenden 8, 9 des Formteils 7 je ein weiteres oder ein in etwa flaches Teil 10, 11 anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann. Das in etwa flache Teil kann jede beliebige Querschnittsform aufweisen, die aber so ausgebildet sein sollte, dass das Gesichtsfeld auf jeden Fall ausreichend begrenzt wird. Die Verbindungselemente zwischen den einzelnen Teilen können elastische Verbindungselemente oder kleine Scharniere sein, die auch mit Federelementen ausgestattet werden können, um sicherzustellen, dass die einzelnen Teile an die Kopfform angedrückt werden.

Vorteilhaft ist es auch, dass an beiden Seitenenden 8, 9 des Abstützteils 7 je ein in etwa flaches Teil 10, 11 anschließt, das mit Bezug auf die Standfläche des Menschen horizontal verläuft oder zumindest horizontal oder in etwa horizontal einstellbar ist. Die Verstellmöglichkeit des flachen Teils stellt sicher, dass es immer so ausgerichtet werden kann, dass ein kleiner Bereich der Standfläche des Menschen nicht wahrgenommen werden kann.

Ferner ist es möglich, dassdas flache und/oder horizontal einstellbare Teil 10, 11, das beiderseits des abstützbaren Teils und/oder des Formteils 7 vorgesehen ist, mit dem abstützbaren Teil und/oder Formteil 7 beweglich und/oder einstellbar und/oder lösbar verbunden ist.

Von besonderer Bedeutung ist es auch, dass das flache und/oder horizontal einstellbare Teil 10, 11, das beiderseits des abstützbaren Teils und/oder Formteils 7 vorgesehen ist, mit je einem weiteren seitlich am Kopf verlaufenden Teil 4, 6 beweglich und/oder einstellbar und/oder lösbar verbunden ist. Auf diese Weise erhält man eine sehr gute Anpassung an den Kopfumfang des Spielers, da sich das seitlich am Kopf in etwa vertikal verlaufende Teil an eine seitliche Kopffläche anlegt und/oder auf den Ohren des Kopfes abgestützt werden kann.

Ferner ist es vorteilhaft, dass die vorderen Teile 3-11 des Sichtfeldes eines Menschen verstellbar ausgebildet und/oder miteinander einstellbar verbunden sind, wobei der Querschnitt zumindest einzelner Teile flach, oval oder so ausgebildet sind, dass sie das Sichtfeld des Menschen zumindest zu einer Seite oder in eine Richtung begrenzen, wobei zumindest ein oder mehrere Teile des Tragteils 2 unterhalb der Augen am Kopf des Menschen befestigt werden.

Vorteilhaft ist es auch, dass das am Körper oder am Kopf zu befestigende Tragteil 2 die Kopfform ganz umschließt und der hintere Teil mit Bezug auf den Kopf eines Menschen als einstellbares Teil und/oder elastisch einstellbares und/oder längenveränderliches Teil oder Band ausgebildet ist.

Auch ist es von Vorteil, dass einzelne oder alle Teile des am Kopf zu befestigen Tragteils Einzelelemente sind, die mit dem jeweils benachbarten Teil fest und/oder lösbar und/oder beweglich und/oder einstellbar verbunden sind.

Damit ein noch besserer Halt des Tragteils 2 am Körper oder am Kopf 14 gewährleistet wird, weist das zu befestigende Tragteil 2 ein weiteres Befestigungsteil 16 auf, mit dessen Hilfe das Tragteil 2 noch sicherer am Kopf 14 befestigt werden kann. Das Befestigungsteil 16 ist hierzu an die Seitenteile 6 entweder fest oder beweglich angeschlossen.

### Bezugszeichenliste

- **1**: Vorrichtung
- **2**: Tragteil
- **3**: Teil, Band
- **4**: Teil
- **5**: Teil
- **6**: Teil
- **7**: Formteil, Abstützteil
- **8**: Seitenende
- **9**: Seitenende
- **10**: ein weiteres in etwa flaches Teil
- **11**: ein weiteres in etwa flaches Teil
- **12**: Kopffläche
- **13**: Ohr
- **14**: Kopf
- **16**: Befestigungsteil

## Patentansprüche

1. Vorrichtung (1) zur Begrenzung des Sichtfeldes eines Menschen mit Hilfe eines am Körper oder am Kopf zu befestigenden Tragteils (2) **dadurch gekennzeichnet,**
**dass** das Tragteil (2) eine Einrichtung aufweist, mit deren Hilfe das Sichtfeld des Menschen auf einen Ausschnitt eingeschränkt wird.

2. Vorrichtung nach Anspruch 1,
**dadurch gekennzeichnet,**
**dass** das Tragteil (2) als Band (3) ausgebildet ist, das elastisch und/oder längenveränderlich ausgebildet ist und am Kopf des Menschen befestigt werden kann.

3. Vorrichtung nach Anspruch 1 oder 2,
**dadurch gekennzeichnet,**
**dass** das Tragteil (2) aus einem festen Material besteht, das an Teilen des Kopfes oder an der Nase und/oder an den Ohren (13) abstützbar ist.

4. Vorrichtung nach einem der vorhergehenden Ansprüche,
**dadurch gekennzeichnet,**
**dass** das Tragteil aus einem oder mehreren Teilen (3, 4, 5, 6) besteht, die fest und/oder einteilig miteinander verbunden sind, um es unterschiedlichen Kopfformen anzupassen.

5. Vorrichtung nach einem der vorhergehenden Ansprüche,
**dadurch gekennzeichnet**,
zumindest ein Teil oder das vordere Teil (3) ein sich auf dem Nasenrücken abstützendes Formteil (7) aufweist, an das sich an beiden Seitenenden (8, 9) des Formteils (7) je ein weiteres oder ein in etwa flaches Teil (10, 11) anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann..

6. Vorrichtung nach einem der vorhergehenden Ansprüche,
**dadurch gekennzeichnet,**
**dass** zumindest ein Teil oder das vordere Teil (3) ein sich auf dem Nasenrücken abstützendes Formteil (7) aufweist, an das sich an beiden Seitenenden (8, 9) des Formteils (7) je ein weiteres oder ein in etwa flaches Teil (10, 11) anschließt, sodass das Sichtfeld des Menschen nach unten auf einen bestimmten Bereich begrenzt ist oder so begrenzt werden kann, dass ein kleiner Bereich der Standfläche des Menschen nicht wahr genommen werden kann.

7. Vorrichtung nach einem der vorhergehenden Ansprüche,
**dadurch gekennzeichnet,**
**dass** an beiden Seitenenden (8, 9) des Abstützteils (7) je ein in etwa flaches Teil (10, 11) anschließt, das mit Bezug auf die Standfläche des Menschen horizontal verläuft oder zumindest horizontal einstellbar ist.

8. Vorrichtung nach einem der vorhergehenden Ansprüche,
**dadurch gekennzeichnet,**
**dass** das flache und/oder horizontal einstellbare Teil (10, 11), das beiderseits des abstützbaren Teils und/oder des Formteils (7) vorgesehen ist, mit dem abstützbaren Teil und/oder Formteil (7) beweglich und/oder einstellbar und/oder lösbar verbunden ist.

9. Vorrichtung nach einem der vorhergehenden Ansprüche,
**dadurch gekennzeichnet,**
**dass** das flache und/oder horizontal einstellbare Teil (10, 11), das beiderseits des abstützbaren Teils und/oder Formteils (7) vorgesehen ist, mit je einem weiteren seitlich am Kopf verlaufenden Teil (4, 6) beweglich und/oder einstellbar und/oder lösbar verbunden ist.

10. Vorrichtung nach einem der vorhergehenden Ansprüche,
**dadurch gekennzeichnet,**
**dass** sich das seitlich am Kopf verlaufende Teil (4, 6) an eine seitliche Kopffläche (12) anlegt und/oder auf den Ohren (13) des Kopfes abgestützt werden kann.

11. Vorrichtung nach einem der vorhergehenden Ansprüche,
**dadurch gekennzeichnet,**
**dass** die vorderen Teile (3 -11) des Sichtfeldes eines Menschen verstellbar ausgebildet und/oder miteinander einstellbar verbunden sind, wobei der Querschnitt zumindest einzelner Teile flach, oval oder so ausgebildet sind, dass sie das Sichtfeld des Menschen zumindest zu einer Seite oder in eine Richtung begrenzen, wobei zumindest ein oder mehrere Teile des Tragteils (2) unterhalb der Augen am Kopf des Menschen befestigt werden.

12. Vorrichtung nach einem der vorhergehenden Ansprüche,
**dadurch gekennzeichnet,**
**dass** das am Körper oder am Kopf zu befestigende Tragteil (2) die Kopfform ganz umschließt und der hintere Teil mit Bezug auf den Kopf eines Menschen als einstellbares Teil und/oder elastisch einstellbares und/oder längenveränderliches Teil oder Band ausgebildet ist.

13. Vorrichtung nach einem der vorhergehenden Ansprüche,
**dadurch gekennzeichnet,**
**dass** einzelne oder alle Teile des am Kopf zu befestigen Tragteils (2) Einzelelemente sind, die mit dem jeweils benachbarten Teil fest/oder lösbar und/oder beweglich und/oder einstellbar verbunden sind.

14. Vorrichtung nach einem der vorhergehenden Ansprüche,
**dadurch gekennzeichnet,**
**dass** das am Körper oder am Kopf (14) zu befestigende Tragteil (2) ein weiteres Befestigungsteil (16) aufweist, mit dessren Hilfe das Tragteil noch sicherer am Kopf befestigt werden kann.
